# EUROPEAN PATENT APPLICATION

(11) **EP 3 978 035 A2**
(43) Date of publication of application: **06.04.2022**
(21) Application number: 21200009.5
(22) Date of filing: 29.09.2021
(51) Int. Cl.: A61L 2/22, B05B 1/00, B60S 1/00, B62B 3/00, B64D 11/00, B64F 5/30

(54) **AURA DISINFECTING MACHINE**

(30) Priority: 30.09.2020 SG 10202009736
(71) Applicant: Sureclean Pte. Ltd., Singapore 658079 (SG)
(72) Inventor: TAN HIANG KIAT, Alvin, 649823 Singapore (SG)
(74) Representative: Adamson Jones

(57) **Abstract**

The application provides a disinfecting device. The device comprises an aircraft trolley-sized body comprising two side surfaces. The body is adapted to move along a longitudinal axis of an aisle of an aircraft such that the side surfaces face two opposing sides of the aisle. The device includes a powering unit, at least one air compressor for providing compressed air, a disinfectant bottle for storing liquid disinfectants, and at least two nozzle assemblies. Each nozzle assembly includes an electrically actuated valve, a connector, and at least three nozzles. The compressed air flows from the compressor to the valve, to the connector, and to the nozzles to form droplets of disinfectants. The nozzles are provided on upper surfaces of the body such that the nozzles point away from the respective side surfaces for spraying the disinfectants onto objects located on the two opposing sides of the aisle.

## Description

The present application relates to a disinfecting device. In particular, it relates to a disinfecting device for sanitizing aircraft cabins.

Infectious disease transmission among air travelers is a significant personal and public health concern. Common and potentially serious viral, bacterial, and fungal pathogens are often spread through the air and from mutually contacted surfaces. Commercial aircraft currently use extensive on-board air filtration and can also use chemical disinfectants or ultraviolet "C" band (UVC) technologies to decrease airborne microbes, although other means are also possible.

EP2772272B1 discloses a mobile body that is configured to travel along an aisle inside an aircraft cabin. A source of UV radiation is mounted to the mobile body and configured to direct UV radiation to the surface at a predetermined dosage. At least two articulated arms are also mounted to the mobile body, and UV lamps are mounted respectively on the arms.

It is an object of this application to provide an improved disinfecting device for sanitizing an aircraft.

The application provides a disinfecting device for an aircraft. The aircraft refers to an aircraft. The aircraft has a narrow body having only one aisle or a wide body having two or more aisles. The aisle refers to a long passage. Rows of seats are often located on both sides of the passage. In certain aircrafts, seats are located on one side and a wall is located on the other side of the long passage. The aisle extends in the longitudinal axis of the aircraft.

The disinfecting device is used for sanitizing or to inactivate or destroying microorganisms on cabin interior of the aircraft. The microorganisms can be located on surfaces of wall, seats, and floor and in the air of the aircraft.

The disinfecting device comprises an aircraft trolley-sized body. The body is sized such that it can move along the aisle of the aircraft. The body includes two side surfaces, namely a first vertical side surface and a second vertical side surface, as well as a vertical front surface and a vertical rear surface. A first vertical edge of the front surface is connected to a first vertical edge of the first side surface while a second vertical edge of the first side surface is connected to a first vertical edge of the rear surface. A second vertical edge of the rear surface is connected to a first vertical edge of the second side surface while a second vertical edge of the second side surface is connected to a second vertical edge of the front surface.

The body is adapted to move along a longitudinal axis of the aisle of the aircraft such that the front surface and the rear surface face front and rear of the aircraft and such that the first and the second side surfaces face two opposing sides of the aircraft. In other words, if the first side surface faces the left side of the aisle, the second side surface faces the right side of the aisle, and vice versa.

The disinfecting device further comprise an alternating current (AC) powering unit that is housed or enclosed in the body for providing electrical power in the form of an alternating electrical current.

The disinfecting device also comprises at least one air compressor that is housed in the body for providing compressed air. The air compressor is powered by an alternating electrical current from an AC source, such as the AC powering unit.

The disinfecting device further comprises a disinfectant bottle housed in the body for storing liquid disinfectants. The disinfectants often refer to chemical agents for destroying microorganisms that are on surfaces or are airborne.

The disinfecting device also comprises at least two nozzle assemblies. Each nozzle assembly includes an electrically actuated valve, a multi-port connector, and at least three nozzles.

The electrically actuated valve often refers to a solenoid valve that is energized or actuated by alternating electrical current. The valve is housed in the body. The valve is connected to the air compressor for receiving the compressed air from the air compressor.

The multi-port connector is housed in the body. The multi-port connector has an inlet and several outlets. The inlet is connected to the electrically actuated valve for receiving the compressed air from the electrically actuated valve. The outlets act to receive the compressed air from the inlet and transmit the received compressed air away from the multi-port connector. The multi-port connector having four ports is also called four-way manifold.

The nozzles are respectively connected to the corresponding outlets of the multi-port connector and they act to receive the compressed air from the multi-port connector. The received compressed air creates a vacuum that acts to draw the liquid disinfectant from the disinfectant bottle. The compressed air then mixes with the liquid disinfectants to form numerous droplets of the liquid disinfectants.

The nozzles are provided on upper surfaces of the body such that the nozzles point away from the respective side surfaces towards the left side and the right of the aisle when the body moves along the longitudinal axis of the aisle. The nozzles are intended for spraying the droplets of liquid disinfectants onto surfaces of objects that are located on the two opposing sides, namely the left side and the right side, of the aisle to disinfect the surfaces of the objects. The objects include, for examples, overhead compartment bin covers and their associating latches, seats, seat pull out trays, and window side walls of the aircraft. The objects may be located within approximately two meters of the nozzles.

The disinfecting device can effectively disinfect cabin interior of the aircraft within a short time. This is because the device can disinfect objects located on the left side and on the right side of the aisle together at the same time. This saves time and cost for disinfecting the whole cabin of the aircraft. This is different from disinfecting one side of the aisle before disinfecting another side of the aisle.

Moreover, the user does not need to carry the disinfecting device as it can be moved by pushing it. This different from disinfecting devices that are intended to be carried by a user using a shoulder strap or handle.

The nozzles can be arranged such that they point in different predetermined directions to provide a predetermined fan-shaped spray coverage. The fan-shape refers to a shape of a fan that is made of supporting sticks radiating outwards. The predetermined directions can be selectively altered to allow a further predetermined fan-shaped spray coverage to be provided according to positions and orientations of surfaces of objects that are to be sanitized.

The disinfecting device can also include a pressure adjusting mechanism for adjusting the pressure of the air compressor by a user to achieve a predetermined pressure level. The achieved predetermined pressure level allows the droplets of the liquid disinfectants to be sprayed at a predetermined flow rate through apertures of the nozzles. When the predetermined pressure level is adjusted higher, the predetermined flow rate increases. When the predetermined pressure level is adjusted lower, the predetermined flow rate decreases. This feature allows the user to sanitize surfaces of the objects at a predetermined flow rate according to sizes and positions of the objects, thereby achieving higher sanitization efficiency. Furthermore, the predetermined flow rate of the spray also provides a consistent spray that contains substantially same amount of the disinfectants throughout the sanitization process, thereby achieving essentially consistent sanitization coverage of the surfaces of the objects.

In one implementation, the mechanism refers to a pressure regulator. The pressure regulator includes a knob that is accessible to a user. The user can turn the knob either in clockwise direction or in anticlockwise direction to increase or decrease the pressure of the air compressor.

The AC powering unit can include a battery for storing electrical energy with an inverter connected electrically to the battery. The inverter acts to convert the electrical energy from the battery into a signal with alternating electrical current. The AC powering unit allows the disinfecting device to be used in enclosed areas where AC power sources are not available.

In one implementation, the disinfecting device further comprise a power inlet for receiving electrical power from an external AC power feeding device. This disinfecting device further comprises a power source selector switch for selectively connecting the powering unit or connecting the external AC power feeding device to parts of the disinfecting device for powering the parts of the disinfecting device. This allows the disinfecting device to operate using an ac power source when it is available, thereby extending the operating life of the battery of the disinfecting device.

The aircraft trolley-sized body can comprise a vertical protruding portion. The nozzles are provided on upper surfaces of the vertical protruding portion so that nozzles are positioned at high vertical level. This allows the nozzles to spray the droplets of liquid disinfectants onto objects that are located further away from the nozzles or at a greater height.

The disinfecting device can further comprise a first user controllable switch for allowing the air compressor to receive the electrical power for energizing the air compressor.

The disinfecting device can further comprise a second user controllable switch for allowing the electrically actuated valve of one of the nozzle assemblies to receive the electrical power for energizing the electrically actuated valve of the one of the nozzle assemblies.

The disinfecting device can further comprise a third user controllable switch for allowing the electrically actuated valve of the other nozzle assembly to receive the electrical power for energizing the electrically actuated valve of the other nozzle assembly.
- Fig. 1: illustrates a perspective of an aura disinfecting device 1,
- Fig. 2: illustrates a front view of the aura disinfecting device 1 of Fig. 1,
- Fig. 3: illustrates a side view of the aura disinfecting device 1 of Fig. 1,
- Fig. 4: illustrates a rear view of the aura disinfecting device 1 of Fig. 1,
- Fig. 5: illustrates a schematic block diagram of components of the aura disinfecting device 1 of Fig. 1,
- Fig. 6: illustrates a horizontal cross section view of a part of an aircraft, where the aura disinfecting device 1 of Fig. 1 is positioned to spray liquid disinfectant, and
- Fig. 7: illustrates a horizontal cross section view of another part of the aircraft of Fig. 6, where the aura disinfecting device 1 of Fig. 1 is positioned to spray the liquid disinfectant.

In the following description, details are provided to describe embodiments of the application. It shall be apparent to one skilled in the art, however, that the embodiments may be practiced without such details.

Some parts of the embodiment have similar parts. The similar parts may have the same names or similar part numbers. The description of one similar part also applies by reference to another similar parts, where appropriate, thereby reducing repetition of text without limiting the disclosure.

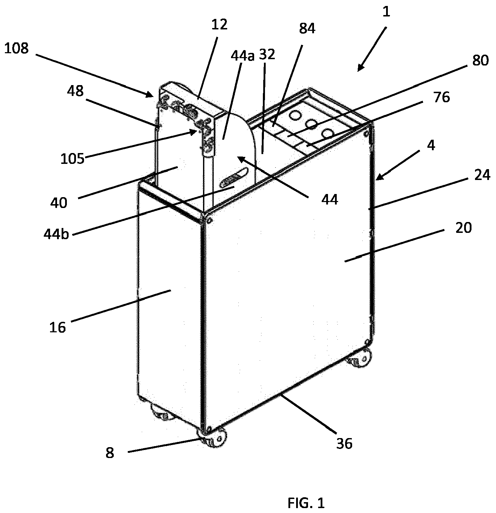

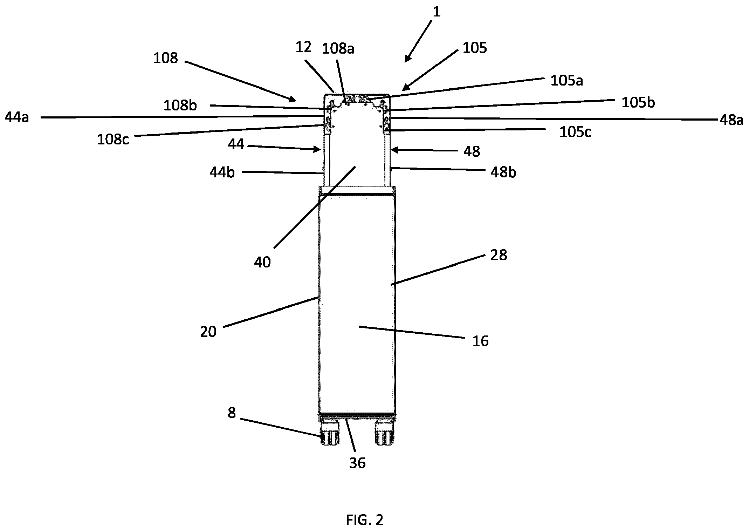

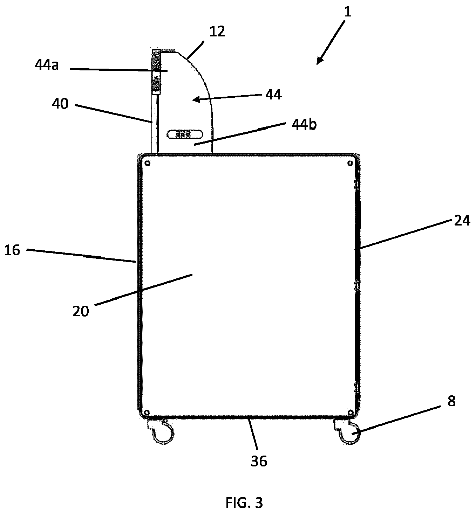

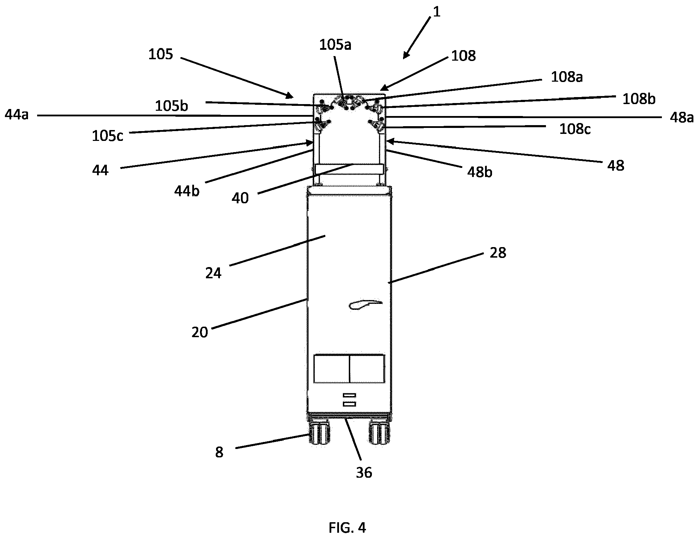

Figs. 1, 2, 3, and 4 show an aura disinfecting device 1 1 for sanitizing cabin interior surfaces of a narrow-body aircraft.

The disinfecting device 1 includes a movable or mobile aircraft trolley-sized body 4 with four twin wheels castors 8 and with a vertical protruding portion 12. The twin wheels castors 8 are attached to a bottom surface of the body 4 while the protruding portion 12 is connected to the body 4 such that the protruding portion 12 is placed above the body 4.

The body 4 has a shape of a rectangular cuboid that includes a vertical front panel 16, a first vertical side panel, a vertical rear panel 24, a second vertical side panel, a horizontal top panel 32, and a horizontal bottom panel 36. A first vertical edge of the front panel 16 is connected to a first vertical edge of the first side panel 20 while a second vertical edge of the first side panel 20 is connected to a first vertical edge of the rear panel 24. A second vertical edge of the rear panel 24 is connected to a first vertical edge of the second side panel 28 while a second vertical edge of the second side panel 28 is connected to a second vertical edge of the front panel 16. The top panel 32 is placed on top of the vertical front panel 16, the vertical rear panel 24, the first vertical side panel, and the second vertical side panel such that all horizontal edges of the top panel 32 are respectively connected to a first horizontal edge of the vertical front panel 16, of the first vertical side panel, of the second vertical side panel, and of the vertical rear panel 24. Similarly, the bottom panel 36 is placed below the vertical front panel 16, the vertical rear panel 24, the first vertical side panel, and the second vertical side panel such that all horizontal edges of the bottom panel 36 are respectively connected to a second horizontal edge of the vertical front panel 16, of the first vertical side panel, of the second vertical side panel, and of the vertical rear panel 24, wherein the second horizontal edge is opposite the first horizontal edge.

In one implementation, the vertical front panel 16 and the vertical rear panel 24 respectively has a dimension of 300 millimeters (mm) in width and 1030 mm in height. The vertical side panels respectively have a dimension of 1030 mm in height and 810 mm in length. The horizontal top panel 32 and the horizontal bottom panel 36 respectively has a dimension of 300 mm in width and 810 mm in length. The panels are configured to be removable to allow access to parts inside the body 4 by a user.

The mobile body 4 further comprises a supporting frame structure that is configured to be slidably positioned inside the aircraft trolley-sized body 4. The supporting frame structure is configured to receive parts of the disinfecting device 1.

The vertical protruding portion 12 includes a vertical front surface 40, a first vertical side surface 44, and a second vertical side surface 48. A first vertical edge of the vertical front surface 40 is connected to a first vertical edge of the first vertical side surface 44 while a second vertical edge of the vertical front surface 40 is connected to a second vertical edge of the second vertical side surface 48.

The front surface 40 is connected to the front panel 16 of the mobile body 4 such that the front surface 40 and the front panel 16 of the mobile body 4 face towards in a same first direction. The first vertical side surface 44 is connected to the first vertical side panel 20 of the body 4 such that the first vertical side surface 44 and the first vertical side panel 20 of the body 4 face towards in a same second direction. The second vertical side surface 48 is connected to the second vertical side panel 28 of the body 4 such that the second vertical side surface 48 and the second vertical side panel 28 of the body 4 face towards in a same third direction.

The first side surface 44 includes a first upper surface portion 44a and a first lower surface portion 44b, which is connected to a lower part of the first upper surface portion 44a. The second side surface 48 includes a second upper surface portion 48a and a second lower surface portion 48b, which is connected to a lower part of the second upper surface portion 48a. An extended part of the second upper surface portion 48a is connected to an extended part of the first upper surface portion 44a.

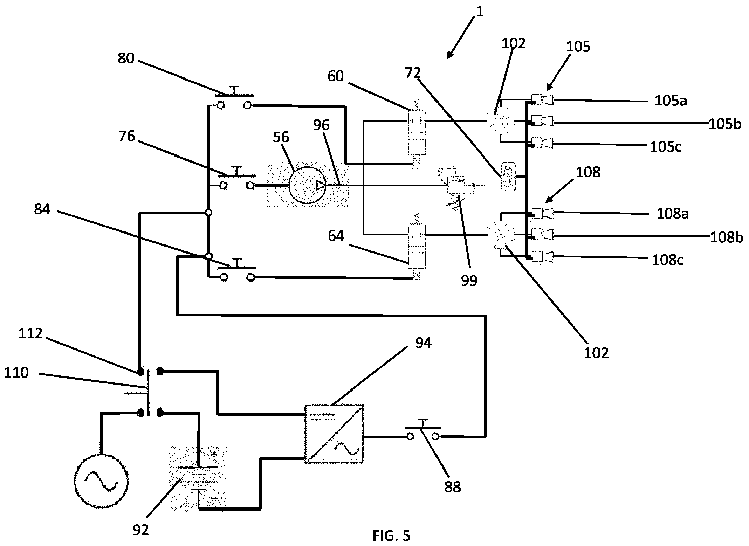

As better seen in Fig. 5, the disinfecting device 1 further includes an internal alternating current (AC) powering unit 52, an AC driven air compressor 56, a first solenoid valve 60, a second solenoid valve 64, two nozzle units 68, a disinfectant bottle 72, a first push button switch 76, a second push button switch 80, a third push button switch 84, and a fourth push button switch 88. The internal AC powering unit 52, the air compressor 56, the solenoid valves 60, 64, the nozzle units 68, and the disinfectant bottle 72 are housed within the aircraft trolley-sized body 4. The first push button switch 76, the second push button switch 80, the third push button switch 84, and the fourth push button switch 88 are mounted on the top panel 32 of the aircraft trolley-sized body 4.

The AC powering unit 52 is connected electrically to the fourth push button switch 88, which is electrically connected to the first push button switch 76, to the second push button switch 80, and to the third push button switch 84. The first push-button switch 76 is electrically connected to the air compressor 56. The second push button switch 80 is connected electrically to the first solenoid valve 60 while the third push button switch 84 is connected electrically to the second solenoid valve 64.

The air compressor 56 is connected pneumatically to both the solenoid valves 60, 64, which are respectively connected pneumatically to the respective corresponding nozzle units 68. The nozzle units 68 are connected to the disinfectant bottle 72.

The AC powering unit 52 includes a battery 92 with a power inverter 94 that is connected to the battery 92. The inverter 94 is electrically connected to the fourth push button switch 88.

The AC compressor includes an air outlet which is connected to a compressor pneumatical line 96 that is connected to a pressure relief valve 99. The compressor pneumatical line 96 is also connected to the solenoid valves 60, 64 via a t-shaped pneumatic joint, which is not shown in Fig. 5 for the reason of simplicity. The pneumatic joint includes a first end opening, a second end opening, and a third end opening. The first end opening is connected to the first solenoid valve 60s and the second end opening is connected to the second solenoid valve 64. The third end opening is connected to the compressor pneumatical line 96.

The solenoid valves 60, 64 are electrically actuated by the alternating current.

Each nozzle units 68 includes a four-way manifold 102 and a set of three spraying nozzles 105, 108, which are connected to the four-way manifold 102. The four-way manifold 102 includes a body with an inlet and three outlets. The inlet is connected to the corresponding solenoid valve 60, 64 while the outlets are connected to the set of the three spraying nozzles 105, 108. Each spraying nozzle is configured to covert liquid that is mixed with air into numerous droplets which have sizes of about 10 micromillimeters, and to project the droplets, in the form a fan shaped spraying pattern, to reach areas or objects that are meters away from the nozzle. The spraying nozzle is also called air atomizing nozzle.

The two sets of the nozzles 105, 108 are mounted on the upper surface portions 44a and 48a of the vertical protruding portion 12. A first set of the nozzles 105 is positioned on the first upper side portion 44a near the first vertical edge of the first side surface 44 while a second set of the nozzles 108 is positioned on the second upper surface portion 48a near the second vertical edge of the second side surface 48. The first set of the nozzles 105 is connected to the first solenoid valve 60 while the second set of the nozzles 108 is connected to the second solenoid valve 64.

Each set of the nozzles 105, 108 include a top nozzle 105a, 108a, a middle nozzle 105b, 108b, and a bottom nozzle 105c, 108c. The top nozzle 105a, 108a is positioned above the middle nozzle 105b, 108b that is positioned above the bottom nozzle 105c, 108c such that the middle nozzle 105c, 108b is separated from the top nozzle 105a, 108a and from the bottom nozzle 105c, 108c with a predetermined distance.

The top nozzles 105a, 108a respectively point outward in an upward direction that is inclined at an angle of about sixty degrees relative to a horizontal plane. The middle nozzles 105b, 108b respectively point outward in a downward direction that is inclined at an angle of about fifteen degrees relative to the horizontal plane. The bottom nozzles 105c, 108c respectively point outward in a downward direction that is inclined at an angle of about forty-five degrees relative to the horizontal plane. The positions and the orientations of these nozzles 105, 108 enable the nozzles 105, 108 to produce a predetermined fan shape coverage of the spray volume.

Orientations of the top nozzles 105a, 108a, of the middle nozzles 105b, 108b, and of the bottom nozzles 105c, 108c can be selectively adjusted such that they point in different directions. This can provide a different fan shape coverage according to positions and orientations of surfaces of objects that are to be sanitized. Put differently, this fan shape coverage is appropriate for the objects that are to be sanitized.

The disinfectant bottle 72 is connected to all the spraying nozzles 105, 108.

The disinfecting device 1 further includes a power inlet 112 and a two-pole switch 110, which is connected to the power inlet 112 and to the battery 92.

The power inlet 112 is connected to the first push button switch 76, to the second push button switch 80, and to the third push button switch 84. The power inlet is configured to connect an electrical able that is connected to an external AC power feeding device, such as an AC power source.

The two-pole switch 110 is configured to be selectively connected electrically to the AC power feeding device that is connected to the power inlet or be selectively connected electrically to the battery 92.

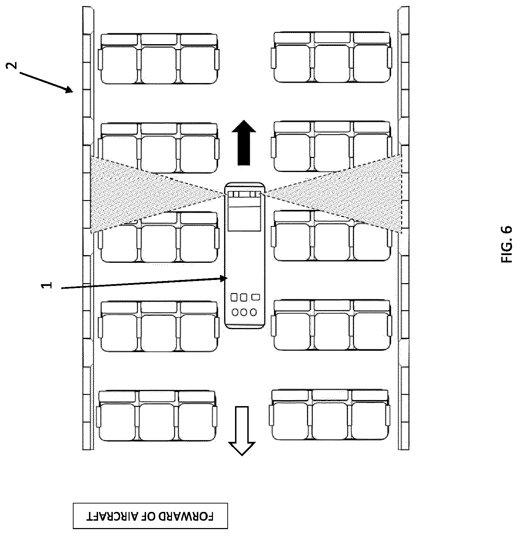

In use, a user pushes or moves the disinfecting device 1 along an aisle of an aircraft 2, as shown in Fig. 6. The twin wheels castors 8 roll over a surface leaving nearly no mark on the surface.

The user then presses the fourth push button switch 88 and then the first push button switch 76, thereby allowing the inverter 94 to connect the air compressor 56. The inverter 94 later receives a direct current from the battery 92 that provides electrical power. The inverter 94 afterward coverts the direct current into an alternating current, which later flows to the air compressor 56 to energize the air compressor 56.

The energized air compressor 56 then generates compressed air which later flows to the compressor pneumatical line 96 and to the solenoid valves 60, 64. The solenoid valves 60, 64 that are initially not powered are placed in open positions. In the open positions, the solenoid valves 60, 64 block the compressed air, thereby allowing the pressure in the compressor pneumatical line 96 to increase. When the pressure exceeds a predetermined level, the pressure relief valve 99 allows air to be automatically released from the compressor pneumatical line 96, thereby allowing the pressure in the compressor pneumatical line 96 to decrease and maintaining the pressure at about the predetermined level.

The user then presses the second push button switch 80 and the third push button switch 84 to allow the alternating current to flow from the inverter 94 to the solenoid valves 60, 64 to energize the solenoid valves 60, 64. The energized solenoid valves 60, 64 are later placed in closed positions, thereby allowing the compressed air to flow to the four-way manifolds 102.

The compressed air afterward flows from the four-way manifolds 102 to the spraying nozzles 105, 108, which receive liquid disinfectants from the disinfectant bottle 72. The compressed air acts to create a vacuum when the compressed air is injected into the atmosphere through apertures of the nozzles 105, 108. The vacuum causes a pressure difference between the bottle 72 and the nozzles 105, 108. This differential pressure creates a suction force that draws the liquid disinfectants from the bottle 72 to the nozzles 105, 108. The suction force can draw the liquid disinfectants up to 400 millimetre in height.

The compressed air and the liquid disinfectants are later mixed in the nozzles 105, 108 to product numerous fine droplets or particles, thereby atomizing the liquid disinfectants. The compressed air afterward pushes the droplets or atomized liquid disinfectants through apertures of the nozzles 105, 108 to form a fan shaped spraying pattern. The spraying pattern can cover surfaces of overhead compartment bin covers and their associating latches, upper and lower surfaces of seats, surfaces of seat pull out trays, and surfaces of window side walls of the aircraft 2. These surfaces are located on the left side and the right side of the aisles. Put differently, the first set of the nozzles 105, 108 spray the liquid disinfectants towards the left side or the first side of the aisle while the second set of the nozzles 105, 108 spray the liquid disinfectants towards the right side or the second side of the aisle at the same time.

The user can afterward selectively stop the spraying of the liquid disinfectants from either set of the nozzles 105, 108 to disinfect only areas on one of the sides of the aisle. In other words, the user can press the second push button switch 80 again to stop current flowing to the first solenoid valve 60, thereby de-energizing the first solenoid valve 60. The deenergized solenoid valve blocks the compressed air from flowing to the first set of the nozzles 105, 108. The first set of the nozzles 105, 108 then stop spraying to the left side of the aisle while the second set of the nozzles 105, 108 continues spraying to the right side of the aisle.

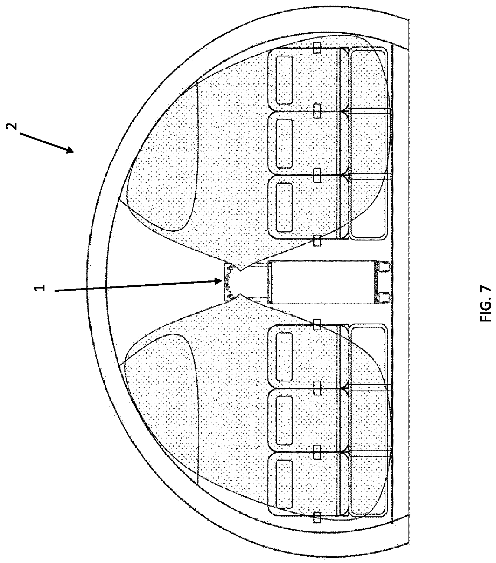

The sanitization of another part of the cabin interior of the aircraft 2 by the disinfecting device 1 is shown in Fig. 7.

In another embodiment, the disinfecting device 1 further comprises a pressure regulator that is connected to the air compressor 56. The pressure regulator includes a knob that is configured to increase or decrease the pressure of the compressed air in the air compressor 56. The knob is provided such that it is accessible to a user. In short, the user can easier reach the knob.

In use, the user can turn the knob, in this example, in clockwise direction to increase the pressure of the compressed air. When the pressure of the compressed air increases, differential pressure between the compressed air in the nozzles 105, 108 and the air outside apertures of the nozzles 105, 108 also increases, thereby increasing the flow rate of the spray of droplets of the liquid disinfectants away from the nozzles 105, 108.

The user can also turn, in this example, in anticlockwise direction to decrease the pressure of the compressed air. When the pressure of the compressed air decreases, the differential pressure then decreases, thereby decreasing the flow rate of the spray of droplets of the liquid disinfectants away from the nozzles 105, 108.

Put differently, the user can turn the knob by a predetermined angular distance to spray the droplets of the liquid disinfectants at a predetermined flow rate. This feature allows the user to sanitize surfaces of the objects at the predetermined flow rate according to sizes and positions of the objects, thereby achieving higher sanitization efficiency. Furthermore, the predetermined flow rate of the spray also provides a consistent spray that contains substantially same amount of the disinfectants throughout the sanitization process, thereby achieving essentially consistent sanitization coverage of the surfaces of the objects.

The number of air compressor 56 is not limited to just one single unit. The number of air compressors can be two or more units according to user requirements.

In one embodiment, each set of the nozzles include four nozzles while in another embodiment, each set of the nozzles include five nozzles.

The arrangement of the nozzles may be selected such that the nozzles point towards in various predetermined directions according to user requirements.

The parts of the disinfecting device 1 have been purposefully selected for improving efficiency and reducing the size of the mobile body 4 that houses the parts of the device 1. In particular, the nozzles with the highest efficiency and lowest power consumption available in the market are selected. The air compressor 56 is selected based on the power consumption of the nozzles and its size. The inverter 94 with a small form factor is also chosen.

The disinfecting device 1 can also be used to sanitize interior of vehicles of Mass Rapid Transit (MRT) or Mass Transit Railway (MTR) system, such as trains. The disinfecting device 1 can also be used for disinfecting interior of marine mass transport vessels such as liners, cruise ships, and ferries, as well as for sanitizing interior of buildings, for examples, offices, hospitals, and schools.

The aura disinfecting device 1 provides several benefits.

The aura disinfecting device 1 is effective to disinfect cabin interior surfaces of an aircraft within a short time. The device 1 can disinfect objects located on the left side and on the right side of the aisle together at the same time while the device 1 is being moved by a single user along the aisle. This is far more efficient than one or more users using an air pressurizing, single nozzle spraying equipment to disinfect the aircraft. Moreover, in the event of deploying workers to a hazard environment, the number of workers can be kept as few as possible.

Although the above description contains much specificity, this should not be construed as limiting the scope of the embodiments but merely providing illustration of the foreseeable embodiments. The above-stated advantages of the embodiments should not be construed especially as limiting the scope of the embodiments but merely to explain possible achievements if the described embodiments are put into practice. Thus, the scope of the embodiments should be determined by the claims and their equivalents, rather than by the examples given.

### REFERENCE NUMBERS

- 1: disinfecting device
- 2: aircraft
- 4: aircraft trolley-sized body
- 8: twin wheels castor
- 12: vertical protruding portion
- 16: front panel
- 20: first side panel
- 24: rear panel
- 28: second side panel
- 32: top panel
- 36: bottom panel
- 40: front surface
- 44: first side surface
- 44a: first upper surface portion
- 44b: first lower surface portion
- 48: second side surface
- 48a: second upper surface portion
- 48b: second lower surface portion
- 52: internal alternating current powering unit
- 56: air compressor
- 60: first solenoid valve
- 64: second solenoid valve
- 68: nozzle unit
- 72: disinfectant bottle
- 76: first push button switch
- 80: second push button switch
- 84: third push button switch
- 88: fourth push button switch
- 92: battery
- 94: inverter
- 96: compressor pneumatical line
- 99: pressure relief valve
- 102: four-way manifold
- 105: first set of nozzles
- 105a: top nozzle
- 105b: middle nozzle
- 105c: bottom nozzle
- 108: second set of nozzles
- 108a: top nozzle
- 108b: middle nozzle
- 108c: bottom nozzle
- 110: two-pole switch
- 112: power inlet

## Claims

1. A disinfecting device for an aircraft comprising
- an aircraft trolley-sized body comprising two side surfaces, the body is adapted to move along a longitudinal axis of an aisle of the aircraft such that the side surfaces face two opposing sides of the aisle,
- a powering unit housed in the body for providing electrical power,
- at least one air compressor housed in the body for providing compressed air,
- a disinfectant bottle housed in the body for storing liquid disinfectants, and
- at least two nozzle assemblies, each nozzle assembly comprising
- an electrically actuated valve housed in the body for receiving the compressed air from the air compressor,
- a connector housed in the body for receiving the compressed air from the electrically actuated valve,
- at least three nozzles for receiving the compressed air from the connector, wherein the compressed air acts to draw the liquid disinfectant from the disinfectant bottle to form droplets of the liquid disinfectants,
wherein the nozzles are provided on upper surfaces of the body such that the nozzles point away from the respective side surfaces for spraying the droplets of liquid disinfectants onto objects that are located on the two opposing sides of the aisle to disinfect the objects.

2. The disinfecting device according to claim 1, wherein the nozzles are arranged such that they point in different predetermined directions.

3. The disinfecting device according to claim 1 or 2 further comprising
a mechanism for adjusting pressure of the air compressor.

4. The disinfecting device according to claim 3, wherein the mechanism comprises a pressure regulator.

5. The disinfecting device according to one of the above-mentioned claims, wherein
the powering unit comprises a battery for storing electrical energy with an inverter connected electrically to the battery for converting the electrical energy into a signal with alternating electrical current.

6. The disinfecting device according to one of the above-mentioned claims further comprising a power inlet for receiving electrical power from an external AC power feeding device.

7. The disinfecting device according to claim 6 further comprising a switch for selectively connecting the powering unit or connecting the external AC power feeding device to parts of the disinfecting device for powering the disinfecting device.

8. The disinfecting device according to one of the above-mentioned claims, wherein
the aircraft trolley-sized body comprises a vertical protruding portion, the nozzles being provided on the vertical protruding portion.

9. The disinfecting device according to one of the above-mentioned claims further comprising
a first user controllable switch for allowing the air compressor to receive the electrical power for energizing the air compressor.

10. The disinfecting device according to one of the above-mentioned claims further comprising
a second user controllable switch for allowing the electrically actuated valve of one of the nozzle assemblies to receive the electrical power for energizing the electrically actuated valve of the one of the nozzle assemblies.

11. The disinfecting device according to claim 10 further comprising
a third user controllable switch for allowing the electrically actuated valve of the other nozzle assembly to receive the electrical power for energizing the electrically actuated valve of the other nozzle assembly.
